Europäisches Patentamt

European Patent Office

Office européen des brevets

(19)

(11) Publication number: 0 400 364
A2

## (12) EUROPEAN PATENT APPLICATION

(21) Application number: 90108708.0

(22) Date of filing: 09.05.90

(51) Int. Cl.5: C08B 37/08, A61K 31/73

(30) Priority: 17.05.89 IT 2053989

(43) Date of publication of application:
05.12.90 Bulletin 90/49

(84) Designated Contracting States:
AT BE CH DE DK ES FR GB GR IT LI LU NL SE

(71) Applicant: FARHISPANIA S.A.
1 de Mayo s/n
Montmelo, Barcelona(ES)

(72) Inventor: Butelman, Federico
Viale Bianca Maria, 19
I-20122 Milan(IT)

(74) Representative: Gervasi, Gemma, Dr. et al
Studio Brevetti e Marchi NOTARBARTOLO &
GERVASI 33, Viale Bianca Maria
I-20122 Milano(IT)

(54) New process for preparing chitosan and its derivatives containing quaternary ammonium groups.

(57) Chitosan and its derivatives containing quaternary ammonium groups are prepared by directly treating waste material from the processing of anthropods with saturated steam under pressure in the presence of an alkaline agent, purifying the chitosan obtained in this manner and subjecting it to a quaternization reaction by treatment with alkylating agents containing ammonium groups.

EP 0 400 364 A2

# NEW PROCESS FOR PREPARING CHITOSAN AND ITS DERIVATIVES CONTAINING QUATERNARY AMMO-NIUM GROUPS

## Field of the invention

This invention relates to the preparation of chitosan and its derivatives suitable for use as dietetic fibre and as hypocholesteremic agents respectively.

## Prior art

Processes are known for preparing chitosan and its derivatives containing quaternary ammonium groups starting from anthropod skeleton residues

Chitosan has a known alimentary use as a dietetic fibre and its derivatives a known pharmacological use as hypocholesteremic agents.

The known chitosan preparation processes are mostly conducted in heterogeneous phase operating generally in the following manner.

Skeleton residues of anthropods of various origins are firstly ground, then treated with hydrochloric acid to eliminate inorganic salts, washed, and then treated under hot conditions with dilute NaOH solutions to eliminate proteins and colouring matter.

After repeated washing with water and organic solvents chitin is obtained, this then being treated with a NaOH solution of approximately 40% concentration by weight at a temperature of 100-120° C for 20-40 hours.

By means of this treatment the chitin is partly N-deacetylated to obtain chitosan, which is repeatedly washed.

The chitosan obtained in this manner has a block copolymer structure consisting of irregularly distributed N-polyacetylglucosamine and polyglucosamine sequences in which the non-deacetylated macromolecules (about 20%) represent difficultly accessible zones.

The molecular weight of this commercially available chitosan generally exceeds 500,000 ($[\eta]$ = 12-18 dl/g), and for use as a dietetic fibre and a raw material for hypocholesteremic derivatives it must be further depolymerized with nitrous acid, with the drawback of a further increase in the non-deacetylated component.

## Summary of the invention

We have now discovered a new process for the preparation of chitosan and its derivatives containing quaternary ammonium groups which has technical and commercial advantages compared with known processes.

Said process is characterised in that:

a) waste material from the processing of anthropods is treated directly with saturated steam under pressure in the presence of an alkaline agent;

b) the pressure is released instantaneously;

c) the solid part consisting of chitosan and inorganic salts is recovered by filtration;

d) the inorganic salts are eliminated by adding an acid solution, followed by neutralization with a basic solution and washing with water;

e) if desired, the chitosan can be subjected to a quaternization reaction by treatment with alkylating agents containing ammonium groups.

In a single step (step a), this process enables a type of chitosan to be obtained which is completely deacetylated and has a large specific surface such as to completely satisfy the requirements for use as dietetic fibre and as raw material for the preparation of derivatives containing ammonium groups for use as hypocholesteremic agents.

## Detailed description of the invention

The characteristics and advantages of the process according to the present invention will be apparent from the following detailed description.

The raw material used consists of shells deriving from the processing of crustaceans, to which an aqueous 40-45 wt% caustic soda solution or a mixture of an aqueous 2-10 wt% caustic soda solution and dimethylacetamide in a ratio of between 10:90 and 50:50 is added.

The weight ratio of NaOH to said shells is between 0.05 and 1.

The mixture is treated with saturated steam at a temperature of between 150° C and 200° C at a pressure of between 15 and 30 bar for a time of between 2 and 15 minutes.

On termination of this treatment the pressure is discharged instantaneously.

We have found that the described treatment produces complete disgregation of the starting material, complete depolymerization of the protein component, degradation of the colouring matter and N-deacetylation and depolymerization of the chitosan.

This complete N-deacetylation together with the instantaneous depressurization of the reactor results in a substantial chemical and morphological modification to the chitosan, making it extremely reactive both in interaction with mixed micellar systems (consisting of bile salts, fatty acids, monoglycerides, lecithins and cholesterol) an-in derivative-formation with alkylating agents.

The mixture obtained by the described treatment is filtered to eliminate the liquid part, the solid part then being washed with acid, neutralized by alkaline treatment and washed firstly with water and then with methanol.

In this manner the inorganic salts and degradation products are completely eliminated.

The chitosan obtained has the following characteristics:
- molecular weight between 20,000 and 100,000
- degree of crystallinity less than 40%
- acetyl group content less than 5%
- specific surface (expressed as water retention value) greater than 80 $cm^3/g$.

This type of chitosan is particularly suitable for use as dietetic fibre and in the quaternization reaction, this being effected by treatment with alkylchlorides or with dialkylsulphates or with epoxy compounds containing ammonium groups.

The quaternization reaction is preferably effected by treating the chitosan with glycidyltrimethylammonium chloride in a reaction medium consisting preferably of an 80:20 2-propanol/water mixture, or dimethylacetamide.

The reaction is conducted at a temperature of between 70° C and 180° C for a time of between 15 minutes and 10 hours.

The weight ratio of the chitosan to glycidyltrimethylammonium chloride used in the reaction is between 1:2 and 1:3.

On termination of the reaction the mixture is filtered and the quaternized derivative is washed with methanol and dried under vacuum.

The derivatives obtained have the following characteristics:
- molecular weight between 20,000 and 100,000
- degree of crystallinity less than 30%
- acetyl group content less than 5%
- degree of substitution with ammonium groups between 1.5 and 2
- specific surface (expressed as water retention value) greater than 80 $cm^3/g$.

The derivatives obtained are able to capture large quantities of bile salts in a very short time and have high hypocholesteremic activity.

Either batch or continuous plants can be used in implementing the process of the present invention.

In general, autoclaves of any type can be used, but they must be able to discharge the pressure within a very short time to allow not only a high level of N-deacetylation of the chitin but also its morphological modification.

By virtue of their characteristics the chitosan and its derivatives according to the invention can be formulated with excipients normally used in the pharmaceutical field to obtain pharmaceutical compositions for use as dietetic fibre and as hypocholesteremic agents respectively.

The following examples are given to illustrate the process of the present invention.

EXAMPLE 1

15kg of shells originating from the processing of crustaceans and previously impregnated with 40 litres of a 42% (w/w) NaOH solution are fed into a 400 litre autoclave.

Saturated steam at a temperature of 180° C is then introduced, the material is allowed to react for 6 minutes and is then discharged within a period of 2 seconds into a collection vessel.

After filtration, the residue is washed with 1 N HCl, neutralized with 0.1 N NaOH and then washed with water and methanol. 1.5 kg of product are obtained having the following physico-chemical characteristics:
$[\eta]$ of the 0.1 N $CH_3COOH$/0.2 N NaCl solution = 1.5 dl/g, content of acetyl groups (determined by $^1$H-NMR) = 3.2%, specific surface (determined as water retention value) = 100 $cm^3/g$.

The chitosan obtained (1.0 kg) was dispersed in a 2-propanol:water (80:20) mixture after which 2.5 kg of glycidyltrimethylammonium chloride were added and the mixture heated to 80° C for 8 hours.

After cooling, the product was filtered off, washed with methanol and then dried. The degree of substitution determined by $^1$H-NMR was 1.6.

EXAMPLE 2

15kg of crustacean shells are fed into a 400 litre autoclave followed by 50 litres of DMAc (dimetylacetamide). 25 litres of N NaOH solution (4% w/w) are added to the mixture after which the reaction environment temperature is raised to 170° C by means of saturated steam. After a residence time of 20 minutes the material is discharged within a period of 2 seconds into a collection vessel. After filtration, the residue is washed with water, neutralized with 0.1 N NaOH and then washed with water followed by methanol. 1.3 kg of product are obtained having the following physico-chemical characteristics:
$[\eta]$ of the 0.1 N $CH_3COOH$/0.2 N NaCl solution = 1.0 dl/g, content of acetyl groups (determined by $^1$H-NMR) = 1.5%, specific surface = 110 $cm^3/g$.

The material (1.0 kg) is impregnated with a solution of glycidyltrimethylammonium chloride (2.5 kg) in DMAc (2 1) and fed into the autoclave in which the chitosan preparation was conducted. The mixture is raised to 150°C with saturated steam and the reaction conducted for 10 minutes.

After cooling, the product was recovered by filtration, washed with methanol and then dried.

The degree of substitution determined by $^1$H-NMR was 1.8.

## Claims

1. A process for preparing chitosan and its derivatives containing quaternary ammonium groups, characterised in that:

a) waste material from the processing of anthropods is treated directly with saturated steam under pressure in the presence of an alkaline agent;

b) the pressure is released instantaneously;

c) the solid part consisting of chitosan and inorganic salts is recovered by filtration;

d) the inorganic salts are eliminated by adding an acid solution, followed by neutralization with a basic solution and washing with water;

e) the chitosan can be subjected to a quaternization reaction by treatment with alkylating agents containing ammonium groups.

2. A process as claimed in claim 1, characterised in that said alkaline agent is a 40-45% (w/w) aqueous caustic soda solution.

3. A process as claimed in claim 1, characterised in that said alkaline agent is a 2-10% (w/w) aqueous caustic soda solution mixed with dimethylacetamide.

4. A process as claimed in claims 1 to 3, characterised in that the ratio of NaOH to said waste material from the processing of anthropods is between 0.05:1 and 1.0:1.

5. A process as claimed in claim 1, characterised in that said treatment with saturated steam is conducted at a temperature of between 150°C and 200°C at a pressure of between 15 and 30 bar for a time of between 2 and 15 minutes.

6. A process as claimed in claim 1, characterised in that said quaternization reaction is conducted by treating the chitosan with alkylchlorides or with dialkylsulphates or with epoxy compounds containing ammonium groups.

7. A process as claimed in claim 6, characterised in that said quaternization reaction is conducted by treating the chitosan with glycidyltrimethylammonium chloride in a reaction medium consisting of an 80:20 2-propanol/water mixture or dimethylacetamide at a temperature of between 70°C and 180°C.

8. A process as claimed in claim 7, characterised in that the weight ratio of the chitosan to the glycidyltrimethylammonium chloride used in the reaction is between 1:2 and 1:3.

9. Chitosan having a molecular weight of between 20,000 and 100,000, a degree of crystallinity of less than 40%, a content of acetyl groups of less than 5%, and a specific surface, expressed as water retention value, of greater than 80 cm$^3$/g.

10. Chitosan derivatives containing quaternary ammonium groups and having a molecular weight of between 20,000 and 100,000, a degree of crystallinity of less than 30%, a content of acetyl groups of less than 5%, a degree of substitution with ammonium groups comprised between 1.5 and 2, and a specific surface, expressed as water retention value, of greater than 80 cm$^3$/g.

11. Pharmaceutical compositions comprising the chitosan claimed in claim 9, for use as dietetic fibre.

12. Pharmaceutical compositions comprising the chitosan derivatives claimed in claim 10, for use as hypocholesteremic agents.

CLAIMS FOR THE CONTRACTING STATES: GREECE AND SPAIN

1. A process for preparing chitosan and its derivatives containing quaternary ammonium groups, characterised in that:

a) waste material from the processing of anthropods is treated directly with saturated steam under pressure in the presence of an alkaline agent;

b) the pressure is released instantaneously;

c) the solid part consisting of chitosan and inorganic salts is recovered by filtration;

d) the inorganic salts are eliminated by adding an acid solution, followed by neutralization with a basic solution and washing with water;

e) the chitosan can be subjected to a quaternization reaction by treatment with alkylating agents containing ammonium groups.

2. A process as claimed in claim 1, characterised in that said alkaline agent is a 40-45% (w/w) aqueous caustic soda solution.

3. A process as claimed in claim 1, characterised in that said alkaline agent is a 2-10% (w/w) aqueous caustic soda solution mixed with dimethylacetamide.

4. A process as claimed in claim 1 to 3, characterised in that the ratio of NaOH to said waste material from the processing of anthropods is between 0.05:1 and 1.0:1.

5. A process as claimed in claim 1, characterised in that said treatment with saturated steam is conducted at a temperature of between 150°C and 200°C at a pressure of between 15 and 30 bar for a time of between 2 and 15 minutes.

6. A process as claimed in claim 1, characterised in that said quaternization reaction is conducted by treating the chitosan with alkylchlorides or with dialkylsulphates or with epoxy compounds containing ammonium groups.

7. A process as claimed in claim 6, characterised in that said quaternization reaction is conducted by treating the chitosan with glycidyltrimethylammonium chloride in a reaction medium consisting of an 80:20 2-propanol/water mixture or dimethylacetamide at a temperature of between 70°C and 180°C.

8. A process as claimed in claim 7, characterised in that the weight ratio of the chitosan to the glycidyltrimethylammonium chloride used in the reaction is between 1:2 and 1:3.